(19) Europäisches Patentamt / European Patent Office / Office européen des brevets

(11) **EP 3 449 822 A1**

(12) **EUROPEAN PATENT APPLICATION**
published in accordance with Art. 153(4) EPC

(43) Date of publication:
06.03.2019 Bulletin 2019/10

(51) Int Cl.:
*A61B 5/103* [(2006.01)]     *A61B 8/00* [(2006.01)]
*G06T 7/00* [(2017.01)]

(21) Application number: 16899728.6

(22) Date of filing: 25.04.2016

(86) International application number:
**PCT/CN2016/080159**

(87) International publication number:
**WO 2017/185214 (02.11.2017 Gazette 2017/44)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicant: **Telefield Medical Imaging Limited**
**Hong Kong (CN)**

(72) Inventors:
• **ZHENG, Yongping**
  **Hong Kong (CN)**
• **ZHOU, Guangquan**
  **Hong Kong (CN)**

(74) Representative: **Hellmich, Wolfgang**
**European Patent and Trademark Attorney**
**Lortzingstrasse 9 / 2. Stock**
**81241 München (DE)**

(54) **METHOD AND DEVICE FOR MEASURING SPINAL COLUMN CURVATURE**

(57)     A method and device for measuring spinal column curvature. The method comprises following steps: S1 acquiring a spinal column image by using a three-dimensional ultrasound imaging unit; S2 enhancing, dissecting, and thinning the spinal column image; S3 obtaining, on basis of the enhanced, dissected, and thinned spinal column image, a curve point representing a position of a vertebra; S4 generating a final spinal column curve by using the curve point; S5. and calculating a spinal column curvature angle by using an inflection point of the spinal column curve. The device comprises the three-dimensional ultrasound imaging unit; a processor; and an operating sensor. The method and device use three-dimensional ultrasound imaging to simplify spinal column curvature angle measurement, and use a manual process, a computer-assisted process, or a combination of two processes to collect the curve point, simplify an operation method, and improve precision of a spinal column curvature angle measurement.

S1 acquiring a spinal column image by using three-dimensional ultrasound imaging

↓

S2 enhancing, dissecting and thinning the spinal column image

↓

S3 obtaining, on the basis of the enhanced, dissected and thinned spinal column image, a curve point representing a position of a vertebra

↓

S4 generating a final spinal column curve by using the curve point

↓

S5 calculating a spinal column curvature angle by using an inflection point of the spinal column curve

Fig. 1

**Description**

**FIELD OF THE APPLICATION**

The present application relates to the field of medical measurement technology, and more particularly, relates to a method and device or measuring spinal column curvature applied in the field of medical imaging data processing.

**BACKGROUND OF THE APPLICATION**

The spine consists of twenty-four vertebrae and intervertebral discs, and its normal curvature provides flexibility, elasticity, and shock absorption to the spine. However, scoliosis is a musculoskeletal state in which the spine is abnormally curved, causing the spine to bend left or right. A spine with scoliosis exhibits an S or C letter because of an abnormal curvature. At this time, when the spine cannot develop its normal anterior and posterior arches, the disc may be subjected to abnormal weight. Scoliosis is most common during puberty, where hunchback is a common spinal deformity associated with scoliosis. On a hunchback, the curvature of the spine in the upper back region is forty-five degrees or more, while the normal spine is only about twenty-forty-five degrees in the upper back region. In order to prevent scoliosis or hunchback, it is necessary to measure the spinal column curvature angle of the human body. When the measured spinal column curvature angle is big, it should be corrected in time.

Currently, measuring the spinal column curvature angle is done manually in the lateral X-ray image of the spine. First find the two vertebrae with the largest inclination angle. After selecting these vertebrae, draw a straight line along the upper endplate of the vertebra located at the upper part and the lower endplate of the vertebra located at the lower part. The angle between the two straight lines is the spinal column curvature angle. However, it is difficult to locate the boundary of the vertebrae in the X-ray image of the spine, making it difficult to measure the spinal column curvature angle.

**SUMMARY OF THE APPLICATION**

The technical problem to be solved by the present application is that it is difficult to accurately and conveniently measure the spinal column curvature angle according to the manual calculation in the X-ray image in the prior art, and a method and a device for measuring the spinal column curvature angle are provided.

The technical solution of the present application to solve the above problems is to provide a method for measuring spinal column curvature, wherein, the method includes the following steps:

S1. acquiring a spinal column image by using three-dimensional ultrasound imaging;

S2. enhancing, dissecting and thinning the spinal column image;

S3. obtaining, on the basis of the enhanced, dissected and thinned spinal column image, a curve point representing a position of a vertebra;

S4. generating a final spinal column curve by using the curve point;

S5. calculating a spinal column curvature angle by using an inflection point of the spinal column curve.

In the method for measuring spinal column curvature of the application, in the step S2, the enhancing of the image is achieved by employing symmetric phase consistency of the spine image and obtaining an enhanced spinal bone feature image.

In the method for measuring spinal column curvature of the application, in the step S2, the dissecting of the image is achieved by performing double threshold segmentation on the asymmetric phase consistency of the enhanced spinal bone feature image and the spinal column image to obtain a spine segmentation image including spinous column and transverse processes.

In the method for measuring spinal column curvature of the application, in the step S2, a threshold used for the dissecting of the image is obtained by using a maximum entropy algorithm on the enhanced spinal bone feature image.

In the method for measuring spinal column curvature of the application, in the step S2, the thinning of the image is implemented by performing the following steps from top to bottom or bottom to top on the spine segmentation image:

- find all segmentation lines in the selected row;

- find the segment with the largest overlap with the previous row among all the segmentation lines found, and compare a distance value between the segment and the previous segment with a threshold $T_h$, if the distance value is less than the threshold $T_h$, the segment is taken as a spine bone cross-sectional area of the current row;

- repeat the above steps until all rows have been processed.

In the method for measuring spinal column curvature of the application, in the step S3, the curve point representing the position of the vertebra is realized by finding a position of a minimum region of the gray value of each line of the image in the spine bone cross-sectional area.

In the method for measuring spinal column curvature of the application, in the step S3, including a step of receiving a point marked on the spinal column image provided by an operating sensor and determining it as the obtained curve point representing the position of the vertebra.

In the method for measuring spinal column curvature of the application, in the step S3, including receiving an adjustment provided by the operating sensor for a coordinate position of the curve point representing the position of the vertebra on the spinal column image.

In the method for measuring spinal column curvature of the application, the curve point representing the position of the vertebra is acquired from one or more of the spinal column images.

In the method for measuring spinal column curvature of the application, the curve point representing the position of the vertebra acquired from one or more of the spinal column images is further averaged to obtain the curve point representing the position of the vertebra of the multiple spinal column images.

In the method for measuring spinal column curvature of the application, in the step S4, on the basis of the curve point, a polynomial fitting of 5th order or more is used to generate the final spinal column curve.

In the method for measuring spinal column curvature of the application, the symmetric phase consistency of the spinal column image is acquired in one, two or three dimensions.

In the method for measuring spinal column curvature of the application, the asymmetric phase consistency of the spinal column image is acquired in one, two or three dimensions.

In the method for measuring spinal column curvature of the application, defining the spine range corresponding to the curve before fitting the curve point using a polynomial curve.

In the method for measuring spinal column curvature of the application, in the step S5, the spinal column curvature angle is the maximum angle between the tangents at the inflection point of the curve.

In the device for measuring spinal column curvature of the application, includes a three-dimensional ultrasound imaging unit configured for acquiring a spinal column image by using three-dimensional ultrasound imaging; a processor configured for processing the spinal column image provided by the three-dimensional ultrasound imaging unit; and an operating sensor connected with the processor; the processor includes an enhancing unit configured for enhancing the spinal column image, a dissecting unit configured for dissecting the enhanced image, and a thinning unit configured for thinning the dissected image; a obtaining unit configured for obtaining a curve point representing a position of a vertebra on the basis of the enhanced, dissected and thinned spinal column image; the processor further includes a generating unit configured for generating a final spinal column curve by using the curve point and a calculating unit configured for calculating a spinal column curvature angle by using an inflection point of the spinal column curve.

In the device for measuring spinal column curvature of the application, the enhancing unit includes a device for employing symmetric phase consistency of the spine image and obtaining an enhanced spinal bone feature image; the dissecting unit includes a device for performing double threshold segmentation on the asymmetric phase consistency of the enhanced spinal bone feature image and the spinal column image to obtain a spine segmentation image; the thinning unit includes a device for performing the following steps from top to bottom or bottom to top on the spine segmentation image:

- find all segmentation lines in the selected row;

- find the segment with the largest overlap with the previous row among all the segmentation lines found, and compare a distance value between the segment and the previous segment with a threshold $T_h$, if the distance value is less than the threshold $T_h$, the segment is taken as a spine bone cross-sectional area of the current row;

- repeat the above steps until all rows have been processed.

In the device for measuring spinal column curvature of the application, in the obtaining unit, the curve point representing the position of the vertebra is realized by finding a position of a minimum region of the gray value of each line of the image in the spine bone cross-sectional area.

In the device for measuring spinal column curvature of the application, the obtaining unit includes a device for receiving a point marked on the spinal column image provided by an operating sensor and determining it as the obtained curve point representing the position of the vertebra; the obtaining unit further includes a device receiving an adjustment provided by the operating sensor for a coordinate position of the curve point representing the position of the vertebra on the spinal column image.

In the device for measuring spinal column curvature of the application, the generating unit includes a device for using a polynomial fitting of 5th order or more to generate the final spinal column curve on the basis of the curve point.

The method and device for measuring spinal column curvature of the present application makes it simple and feasible to use three-dimensional ultrasound imaging to measure spinal column curvature angle. Meanwhile, by using a manual process, a computer-assisted process, or a combination of the two processes to collect the curve point, the precision of a spinal column curvature angle measurement is improved, and the entire measurement process is also very convenient and safe.

**BRIEF DESCRIPTION OF THE DRAWINGS**

Figure 1 is a flow chart of a method for measuring spinal column curvature of the present application.

Figure 2 is a flow chart of a method for measuring spinal column curvature according to the first embodiment of the present application.

Figure 3a-3b are graphs showing the results of measuring the spinal column curvature angle according to the first embodiment of the present application.

Figure 4 is a flow chart of a method for measuring spinal column curvature according to the second embodiment of the present application.

Figure 5a-5c are graphs showing the results of measuring the spinal column curvature angle according to the second embodiment of the present application.

Figure 6 is a flow chart of a method for measuring spinal column curvature according to the third embodiment of the present application.

Figure 7a-7c are graphs showing the results of measuring the spinal column curvature angle according to the second embodiment of the present application.

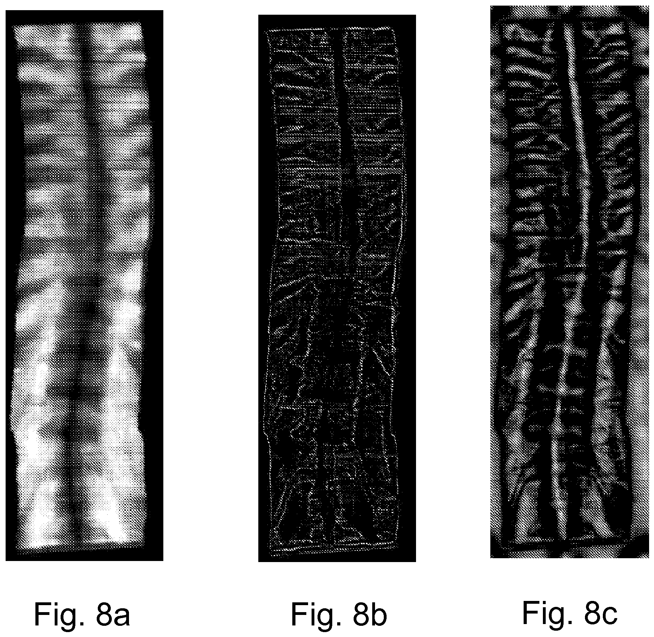

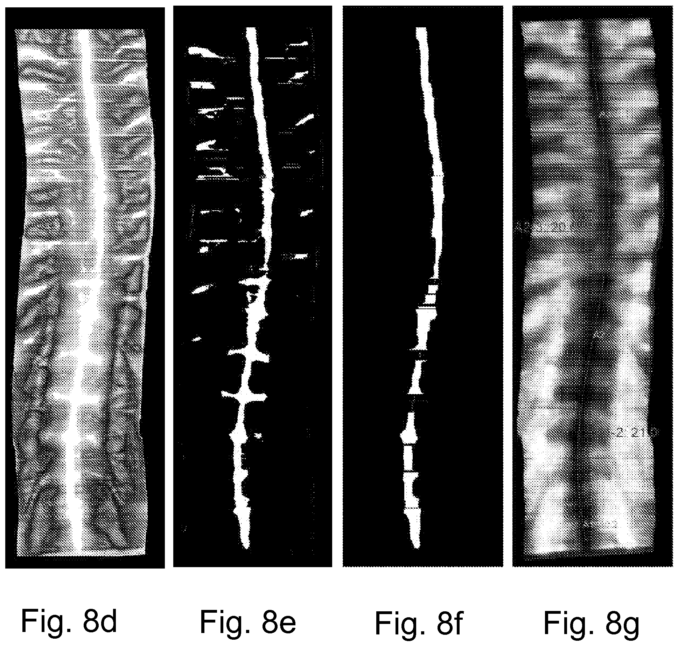

Figure 8a-g are graphs showing the results of measuring the spinal column curvature angle according to the second embodiment of the present application; wherein, Figure 8d is the original spinal column image; Figure 8b is the asymmetric phase consistency image; Figure 8c is the symmetric phase consistency image; Figure 8d is the spine image enhanced with symmetric phase consistency; Figure 8e is the dissected spine image; Figure 8f is a diagram representing the spinous processes of the spine after thinning; Figure 8g is a graph of the measurement results.

Figure 9 is a schematic structural view of an embodiment of a device for measuring spinal column curvature according to the present application.

**DETAILED DESCRIPTION OF THE PREFERRED EMBODIMENT**

The method and device for measuring spinal column curvature of the present application, by performing correlation processing on the spinal column image obtained through three-dimensional ultrasound imaging, a curve point on the spinal column is obtained, and a spinal column curve is formed, thus calculating spinal column curvature angle on the basis of the inflection point on the spinal column curve, making it simple and feasible to use three-dimensional ultrasound imaging to measure spinal column curvature angle. Meanwhile, by using a manual process, a computer-assisted process, or a combination of the two processes to collect the curve point, the needs of different users using 3D imaging systems can be met, thus improving the precision of a spinal column curvature angle measurement.

In order to make the objects, technical solutions and advantages of the present application more comprehensible, the present application will be further described in detail below with reference to the accompanying drawings and embodiments. It is understood that the specific embodiments described herein are merely illustrative of the application and are not intended to limit the application.

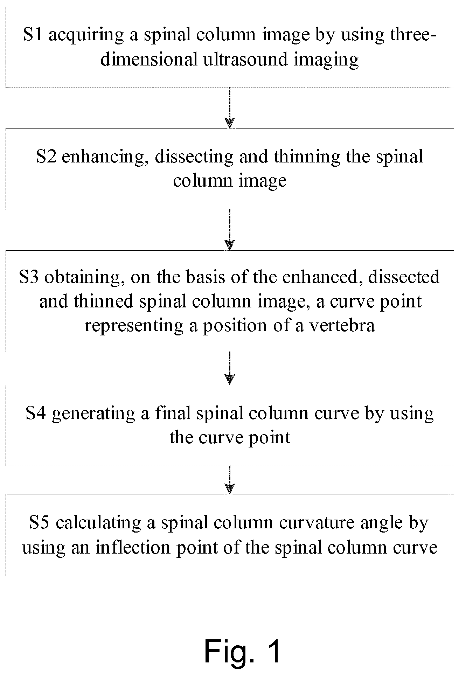

As shown in Figure 1, which is a flow chart of a method for measuring spinal column curvature of the present application, the method includes the following steps:

S1. acquiring a spinal column image by using three-dimensional ultrasound imaging;
In this step, the three-dimensional ultrasound imaging system is used to acquire the spinal column image, and the three-dimensional ultrasound imaging system has been disclosed, and will not be described again here.

S2. enhancing, dissecting and thinning the spinal column image;
Wherein, the enhancing of the image is achieved by employing symmetric phase consistency of the spine image and obtaining an enhanced spinal bone feature image;

The dissecting of the image is achieved by performing double threshold segmentation on the asymmetric phase consistency of the enhanced spinal bone feature image and the spinal column image to obtain a spine segmentation image including spinous column and transverse processes; a threshold used for the dissecting of the image is obtained by using a maximum entropy algorithm on the enhanced spinal bone feature image.

The thinning of the image is implemented by performing the following steps from top to bottom or bottom to top on the spine segmentation image:

- find all segmentation lines in the selected row;

- find the segment with the largest overlap with the previous row among all the segmentation lines found, and compare a distance value between the segment and the previous segment with a threshold $T_h$, if the distance value is less than the threshold $T_h$, the segment is taken as a spine bone cross-sectional area of the current row;

- repeat the above steps until all rows have been processed.

S3. obtaining, on the basis of the enhanced, dissected and thinned spinal column image, a curve point representing a position of a vertebra;
In this step, the curve points located in the spine are obtained by manual and/or computer assisted methods. The specific steps will be described in detail below, and will not be described here.

In the present application, the curve point representing the position of the vertebra is acquired from one spinal column image, or acquired from more spinal column images. The curve points representing the position of the vertebra acquired from more of the spinal column images is first averaged, and the averaged points is taken as the curve point representing the position of the vertebra of the spinal column image.

S4. generating a final spinal column curve by using the curve point;
In this step, on the basis of the curve points, a polynomial fitting of 5th order or more is used to generate the final spinal column curve. Polynomial fitting method belongs to the prior art, and will not be repeated here.

S5. calculating a spinal column curvature angle by using an inflection point of the spinal column curve.

In this step, calculate the angle of the tangent on the inflection point by the coordinates of the inflection point and in combination with the arctangent function. The spinal column curvature angle is the maximum angle between the tangents at the inflection point of the curve.

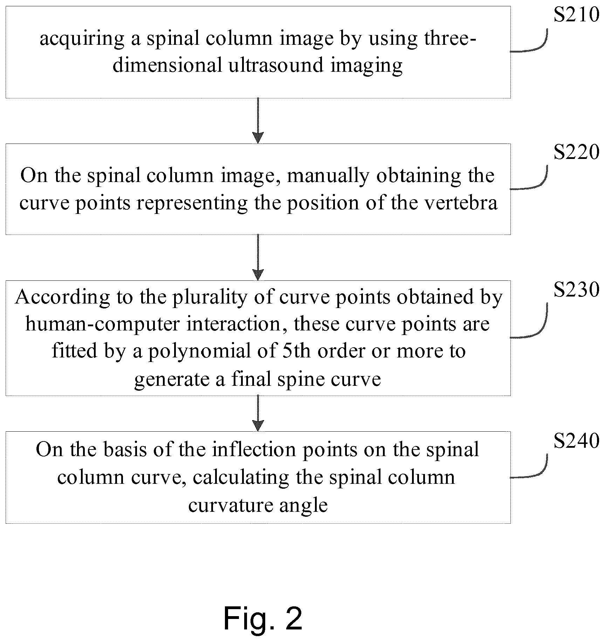

The following are several specific preferred embodiments of the method for measuring spinal column curvature angle of the present application:
As shown in Figure 2, which is a flow chart of a method for measuring spinal column curvature according to the first embodiment of the present application, In this embodiment, the method includes the following steps :

S210. acquiring a spinal column image by using three-dimensional ultrasound imaging;
In this step, the three-dimensional ultrasound imaging system is used to acquire the spinal column image, and the three-dimensional ultrasound imaging system has been disclosed, and will not be described again here.

S220. On the spinal column image, manually obtaining the curve points representing the position of the vertebra;
In this step, the medical staff or the operator of the three-dimensional ultrasound imaging system selects at least 10 curve points according to their own experience, and the curve points are located at the s position of the vertebra. Specifically, the medical staff may display the spinal column image on the image display device by means of a computer interaction device, and one or more curve points are selected by the medical staff through an input device such as a mouse or a touch screen, and the selected curve point coordinate position can be edited. For the system processing, the mark of the medical staff on the spinal column image is received by operational sensing unit, and its position id is determined as the obtained curve point representing the position of the vertebra. On this basis, the coordinate position of the curve point representing the position of the vertebra on the spine image is allowed to be adjusted. Here, you can edit operations such as cutting, inserting, copying, and pasting, and save the position coordinates of these curve points only after confirmation.

S230. According to the plurality of curve points obtained by human-computer interaction, these curve points are fitted by a polynomial of 5th order or more to generate a final spine curve. As an option, the spine range corresponding to the curve can be pre-defined before the polynomial curve is fitted to the plurality of curve points, and the invalid calculation can be reduced.

S240. On the basis of the inflection points on the spinal column curve, calculating the spinal column curvature angle.

In this step, the inflection point is also selected by the medical staff or the operator of the three-dimensional ultrasound imaging system according to their own experience. The angle of the tangent on the inflection point is calculated by the coordinates of the inflection point and in combination with the arctangent function. The spinal column curvature angle is the maximum angle between the tangents at the inflection point of the curve.

In another embodiment, before the step S220, the step S2 of Figure 1 may also be included to enhance, dissect and thin the spinal column image.

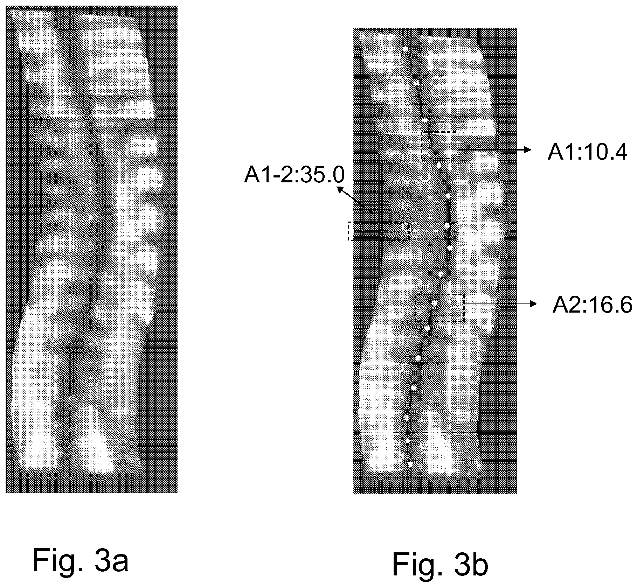

As shown in Figure 3a - 3b, which are graphs showing the results of measuring the spinal column curvature angle according to the first embodiment of the present application, wherein, Figure 3a is the spinal column image acquired by the three-dimensional ultrasound imaging of the first embodiment of the present application, in which there 15 curve points manually obtained by the operational sensing unit; as shown in Figure 3b, on the basis of the inflection points A1 and A2 on the spinal column curve, on which angles of the tangent are 10.4° and 16.6°, respectively, the spinal column curvature angle A1-2 is calculated. Here, manual acquisition can be performed by a pointing device such as a mouse, or by an interactive device such as a touch screen.

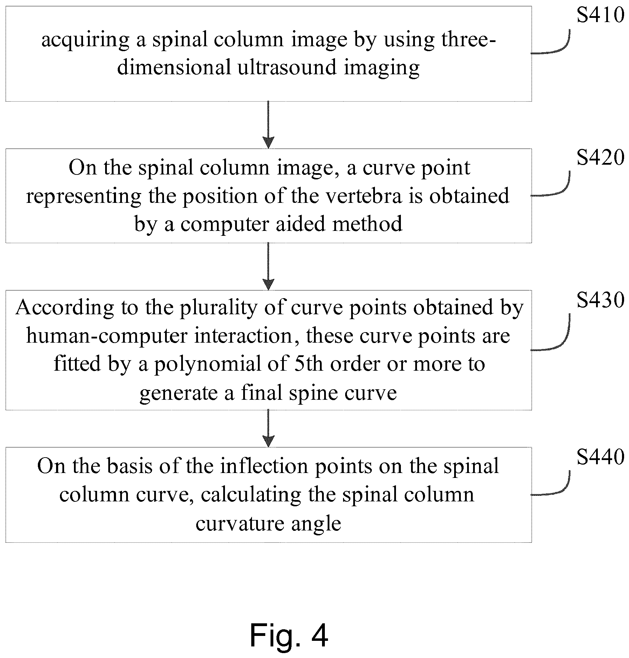

As shown in Figure 4, which is a flow chart of a method for measuring spinal column curvature according to the second embodiment of the present application, the difference between it and the first embodiment is that: a curve point representing the position of the vertebra is obtained by computer-aided method, making it possible to measure the curvature of the vertebra automatically. In this embodiment, the method includes the following steps:

S410. acquiring a spinal column image by using three-dimensional ultrasound imaging;
In this step, the three-dimensional ultrasound imaging system is used to acquire the spinal column image, and the three-dimensional ultrasound imaging system has been disclosed, and will not be described again here.

S420. On the spinal column image, a curve point representing the position of the vertebra is obtained by a computer aided method; specially the following steps are included:

S421. calculating the local phase distribution characteristics of the spinal column image to obtain the phase consistency information of the spinal column image; $Sym(x,y)$ and $Asym(x,y)$;

S422. using the symmetric phase consistency (Phase Congruency) to enhance the spinal column image, and to obtain the enhanced spinal bone feature image $f_e(x,y)$; the symmetric phase consistency here can be presented and calculated in one, two or three dimensions.

S423. using the asymmetric phase consistency and the enhanced image to conduct double threshold segmentation to obtain dissected spinal column image (spinous spine and transverse process);
In this step, specially including:

a. employing OTSU algorithm to dissect the asymmetric phase consistency $Asym(x,y)$ (the asymmetric phase consistency here can be presented in one, two or three dimensions), and obtain a threshold $T_{otsu}$;

b. using the threshold $T_{otsu}$ to dissect spine area:

$$Asym\left(C_{left}^y, y\right) > T_{Otsu}, \text{and } Asym(x, y) \leq T_{Otsu}, x = 1, \cdots, C_{left}^y - 1$$

$$\mathrm{Asym}\left(C_{right}^{y}, y\right) > T_{Otsu}, \text{ and } \mathrm{Asym}(x, y) \leq T_{Otsu}, x = C_{right}^{y} + 1, \cdots, n$$

$$\left|C_{left}^{y} - C_{left}^{y-1}\right| < T_{sp} \text{ and } \left|C_{right}^{y} - C_{right}^{y-1}\right| < T_{sp}$$

    c. within the spine area, using the maximum entropy algorithm on the enhanced image to obtain the threshold $T_{ME}$ used by the dissecting;

    d. within the spine area, using the threshold $T_{ME}$ to dissect the dissected spine (spinous spine and transverse process) image:

$$B(x, y) = \begin{cases} 1 & f_e(x, y) > T_{ME} \\ 0 & \text{otherwise} \end{cases}$$

S424. detecting the spine bone cross-sectional area by line in the direction of the ordinate from the bottom to the top of the dissected image B(x, y);
In this step, specially including:

    a. on the basis of the spine range, detecting the number $N^{y}$ of segments of the y-line segmentation in the image;

    b. if the number of segments is less than 1, perform step d;

    c. find the segment with the largest overlap with the previous row among the $N^{y}$ segmentations, and compare a distance value between the segment and the previous segment with a threshold $T_h$, if the distance value is less than the threshold $T_h$, the segment is taken as a spine bone cross-sectional area of the current row, otherwise perform step d;

    d. judging whether the y line is the last line of the enhanced image $f(x,y)$, and if so, step S425 is performed, otherwise the steps a-b are repeated for the number of lines y+1 according to the above steps..

S425. from the bottom to the top, the point $S_{curve}^{y}(x, y)$ where the gray value of the image in the spine bone cross-sectional area is the smallest in the direction of the ordinate is detected line by line as the curve point on the spine. In the present application, the curve point representing the position of the vertebra is realized by finding a position of a minimum region of the gray value of each line of the image in the spine bone cross-sectional area;

S430. on the basis of the curve point, a polynomial fitting of 5th order or more is used to generate the final spinal column curve;

S440. on the basis of the inflection points on the spinal column curve, calculating the spinal column curvature angle.

In this step, the inflection points on the spinal column curve are determined first. Then, through the coordinates of each inflection point and the inverse tangent function, the angle of the tangent on the inflection point is calculated, wherein the spinal column curvature angle is the maximum angle between the tangent lines on the inflection point of the curve.

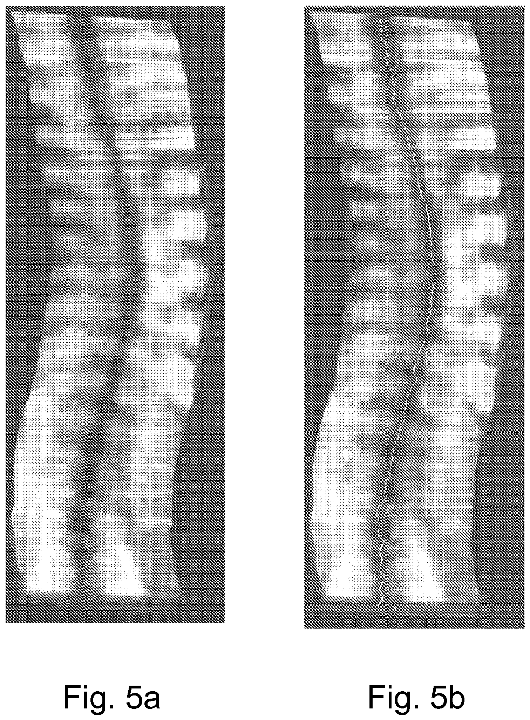

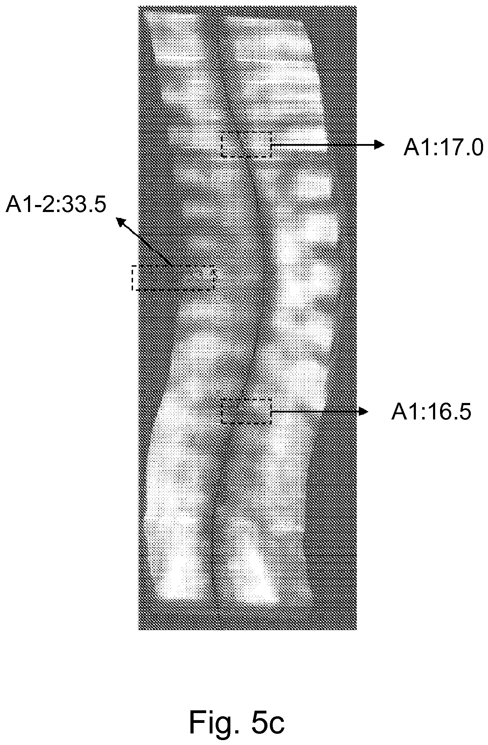

As shown in Figure 5a - 5c, which are graphs showing the results of measuring the spinal column curvature angle according to the second embodiment of the present application, wherein, Figure 5a is the spinal column image acquired by the three-dimensional ultrasound imaging of the second embodiment of the present application, which is the same as the spinal column image obtained through three-dimensional ultrasound imaging according to the first embodiment of the present application. The curve points obtained through the above steps are shown in Figure 5b. According to the angle of the tangent on the inflection points on the spinal column curve, the spinal column curvature angle A1 - 2 is calculated, as shown in Figure 5c, the angles of the tangent on the inflection points A1 and A2 marked in the figure are 17.0° and 16.5°.

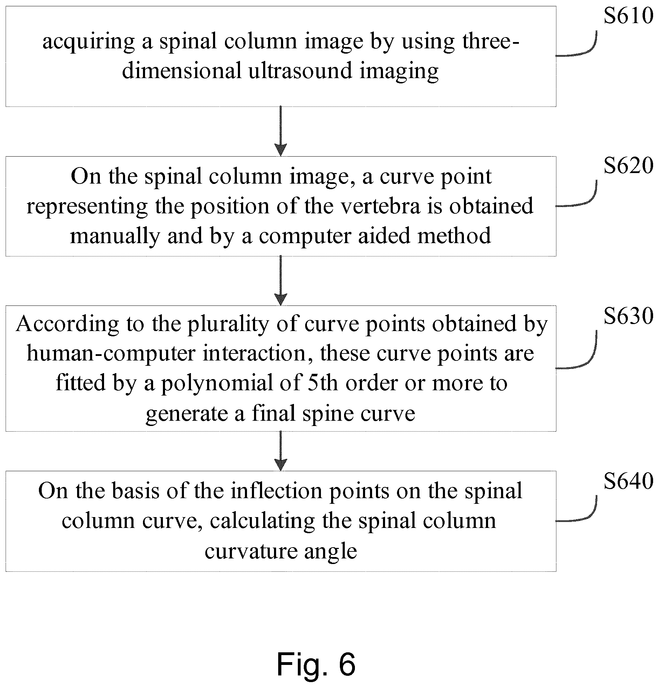

As shown in Figure 6, which is a flow chart of a method for measuring spinal column curvature according to the third embodiment of the present application, the difference between it and the second embodiment is that: not only the computer-aided method but also the manual method are used to obtain the curve points representing the position of the vertebra, so that the measurement of the spinal column curvature angle becomes semi-automatic. The method includes the following steps:

S610. acquiring a spinal column image by using three-dimensional ultrasound imaging;

S620. on the spinal column image, a curve point representing the position of the vertebra is obtained by the operating sensor and the computer aided method, wherein: the number of curve points obtained manually is arbitrary; in the specific step of obtaining the curve points representing the position of the vertebra through computer aided method, when you delete a curve point that does not meet the conditions, the curve points that are manually acquired are included; that is, if the manually obtained curve point does not satisfy the corresponding condition, the corresponding curve point is deleted. The specific steps have been described in detail in the second embodiment, and are not described herein again.

S630. on the basis of the curve point, a polynomial fitting of 5th order or more is used to generate the final spinal column curve;

S640. on the basis of the inflection points on the spinal column curve, calculating the spinal column curvature angle.

In this step, the inflection points on the spinal column curve are determined first. Then, through the coordinates of each inflection point and the inverse tangent function, the angle of the tangent on the inflection point is calculated, wherein the spinal column curvature angle is the maximum angle between the tangents lines on the inflection point of the curve.

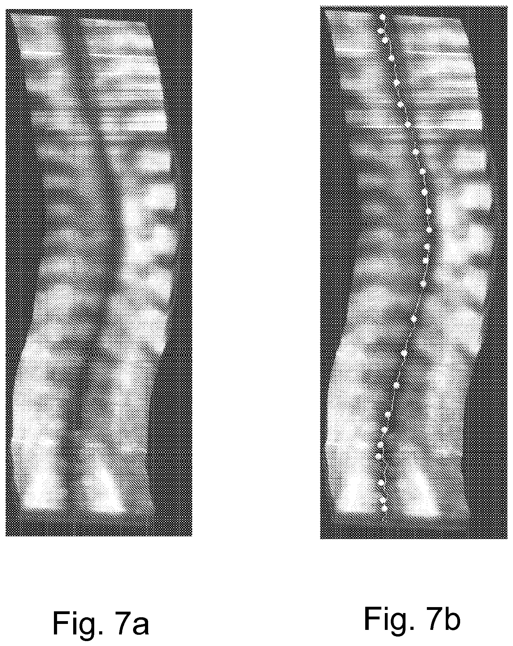

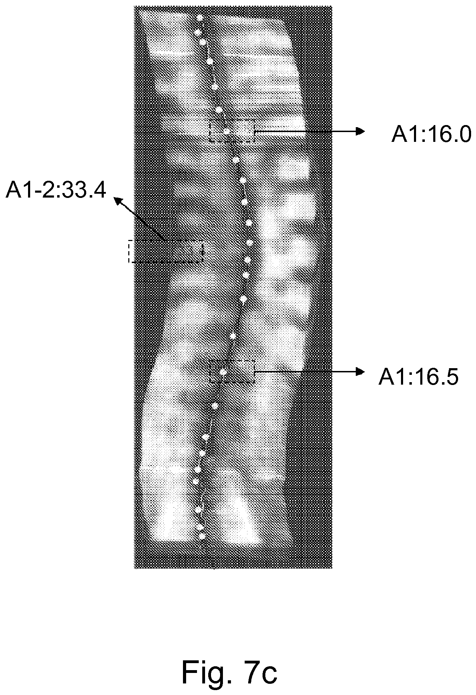

As shown in Figure 7a - 7c, which are graphs showing the results of measuring the spinal column curvature angle according to the third embodiment of the present application, wherein, Figure 7a is the spinal column image acquired by the three-dimensional ultrasound imaging of the third embodiment of the present application, which is the same as the spinal column image obtained through three-dimensional ultrasound imaging according to the first and the second embodiments of the present application. The curve points obtained through the above steps are shown in Figure 7b. According to the angle of the tangent on the inflection points on the spinal column curve, the spinal column curvature angle A1 - 2 is calculated, as shown in Figure 7c, the angles of the tangent on the inflection points A1 and A2 marked in the figure are 16.0° and 16.5°.

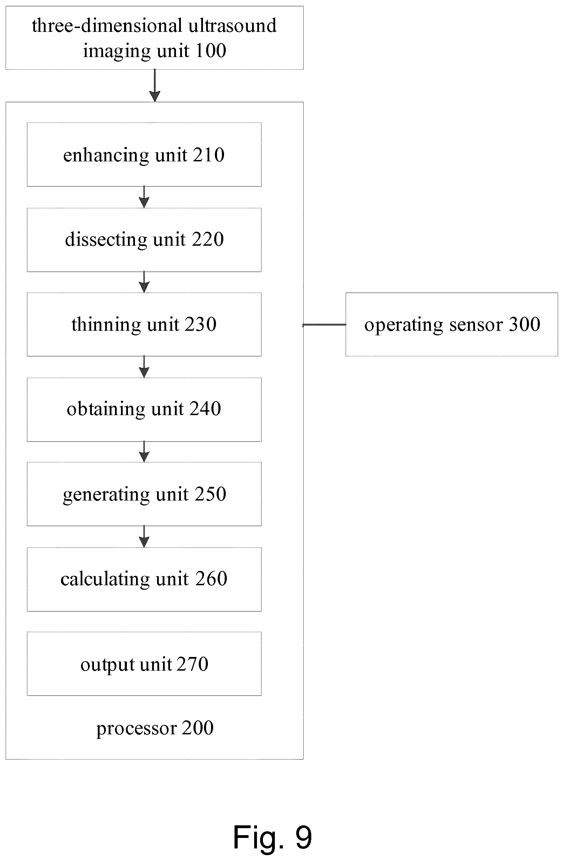

In the schematic structural view of an embodiment of a device for measuring spinal column curvature according to the present application shown in Figure 9, including a three-dimensional ultrasound imaging unit 100 configured for acquiring a spinal column image by using three-dimensional ultrasound imaging; a processor 200 configured for processing the spinal column image provided by the three-dimensional ultrasound imaging unit 100; and an operating sensor300 connected with the processor 200; wherein the processor 200 includes an enhancing unit 210 configured for enhancing the spinal column image, a dissecting unit 220 configured for dissecting the enhanced image from the enhancing unit 210, and a thinning unit 230 configured for thinning the dissected image from the dissecting unit 220; a obtaining unit 240 configured for obtaining a curve point representing a position of a vertebra on the basis of the spinal column image provided by the thinning unit 230; the processor 200 also includes a generating unit 250 configured for generating a final spinal column curve by using the curve point from the obtaining unit 240 and a calculating unit 260 configured for calculating a spinal column curvature angle by using an inflection point of the spinal column curve provided by the generating unit 250; an output unit 270 configured for outputting the results, for example, display, communication output calculation results.

Wherein, the enhancing unit 210 is configured for employing symmetric phase consistency of the spine image to obtain an enhanced spinal bone feature image; the dissecting unit 220 is configured for performing double threshold segmentation on the asymmetric phase consistency of the enhanced spinal bone feature image and the spinal column image to obtain a spine segmentation image; the thinning unit 230 is configured for performing the following steps from top to bottom or bottom to top on the spine segmentation image:

- find all segmentation lines in the selected row;

- find the segment with the largest overlap with the previous row among all the segmentation lines found, and compare a distance value between the segment and the previous segment with a threshold $T_h$, if the distance value is less

than the threshold $T_h$, the segment is taken as a spine bone cross-sectional area of the current row;

- repeat the above steps until all rows have been processed.

Wherein, in the obtaining unit 240, the curve point representing the position of the vertebra is realized by finding a position of a minimum region of the gray value of each line of the image in the spine bone cross-sectional area; or, the obtaining unit 240 is configured for receiving a point marked on the spinal column image provided by an operating sensor and determining it as the obtained curve point representing the position of the vertebra; the obtaining unit 240 is also configured for receiving an adjustment provided by the operating sensor for a coordinate position of the curve point representing the position of the vertebra on the spinal column image.

Wherein, the generating unit 250 is configured for using a polynomial fitting of 5th order or more to generate the final spinal column curve on the basis of the curve point.

The method and device for measuring spinal column curvature of the present application makes it simple and feasible to use three-dimensional ultrasound imaging to measure spinal column curvature angle. Meanwhile, by using a manual process, a computer-assisted process, or a combination of the two processes to collect the curve point, the precision of a spinal column curvature angle measurement is improved. In addition, the method for spinal column curvature of the present application can also utilize a plurality of spine images obtained at different tissue depths to extract curve points and use the same to measure the spinal column curvature angle to improve measurement accuracy.

The above description is only a preferred embodiment of the present application. However, the scope of the present application is not limited thereto, and any changes or substitutions that can be easily conceived within the scope of the present application are within the scope of the present application. Therefore, the scope of protection of the present application should be determined by the scope of protection of the claims.

## Claims

1.  A method for measuring spinal column curvature, wherein, the method includes the following steps:

    S1. acquiring a spinal column image by using three-dimensional ultrasound imaging;
    S2. enhancing, dissecting and thinning the spinal column image;
    S3. obtaining, on the basis of the enhanced, dissected and thinned spinal column image, a curve point representing a position of a vertebra;
    S4. generating a final spinal column curve by using the curve point;
    S5. calculating a spinal column curvature angle by using an inflection point of the spinal column curve.

2.  The method for measuring spinal column curvature according to claim 1, wherein, in the step S2, the enhancing of the image is achieved by employing symmetric phase consistency of the spine image and obtaining an enhanced spinal bone feature image.

3.  The method for measuring spinal column curvature according to claim 1, wherein, in the step S2, the dissecting of the image is achieved by performing double threshold segmentation on the asymmetric phase consistency of the enhanced spinal bone feature image and the spinal column image to obtain a spine segmentation image including spinous column and transverse processes.

4.  The method for measuring spinal column curvature according to claim 3, wherein, in the step S2, a threshold used for the dissecting of the image is obtained by using a maximum entropy algorithm on the enhanced spinal bone feature image.

5.  The method for measuring spinal column curvature according to claim 3, wherein, in the step S2, the thinning of the image is implemented by performing the following steps from top to bottom or bottom to top on the spine segmentation image:

    - find all segmentation lines in the selected row;
    - find the segment with the largest overlap with the previous row among all the segmentation lines found, and compare a distance value between the segment and the previous segment with a threshold $T_h$, if the distance value is less than the threshold $T_h$, the segment is taken as a spine bone cross-sectional area of the current row;
    - repeat the above steps until all rows have been processed.

6. The method for measuring spinal column curvature according to claim 1, wherein, in the step S3, the curve point representing the position of the vertebra is realized by finding a position of a minimum region of the gray value of each line of the image in the spine bone cross-sectional area.

7. The method for measuring spinal column curvature according to claim 1, wherein, in the step S3, including a step of receiving a point marked on the spinal column image provided by an operating sensor and determining it as the obtained curve point representing the position of the vertebra.

8. The method for measuring spinal column curvature according to claim 7, wherein, in the step S3, including receiving an adjustment provided by the operating sensor for a coordinate position of the curve point representing the position of the vertebra on the spinal column image.

9. The method for measuring spinal column curvature according to one of claims 6 - 8, wherein, the curve point representing the position of the vertebra is acquired from one or more of the spinal column images.

10. The method for measuring spinal column curvature according to claim 9, wherein, the curve point representing the position of the vertebra acquired from one or more of the spinal column images is further averaged to obtain the curve point representing the position of the vertebra of the multiple spinal column images.

11. The method for measuring spinal column curvature according to claim 1, wherein, in the step S4, on the basis of the curve point, a polynomial fitting of 5th order or more is used to generate the final spinal column curve.

12. The method for measuring spinal column curvature according to claim 2, wherein, the symmetric phase consistency of the spinal column image is acquired in one, two or three dimensions.

13. The method for measuring spinal column curvature according to claim 3, wherein, the asymmetric phase consistency of the spinal column image is acquired in one, two or three dimensions.

14. The method for measuring spinal column curvature according to claim 11, wherein, defining the spine range corresponding to the curve before fitting the curve point using a polynomial curve.

15. The method for measuring spinal column curvature according to claim 1, wherein, in the step S5, the spinal column curvature angle is the maximum angle between the tangents at the inflection point of the curve.

16. A device for measuring spinal column curvature, wherein, includes a three-dimensional ultrasound imaging unit configured for acquiring a spinal column image by using three-dimensional ultrasound imaging; a processor configured for processing the spinal column image provided by the three-dimensional ultrasound imaging unit; and an operating sensor connected with the processor; the processor includes an enhancing unit configured for enhancing the spinal column image, a dissecting unit configured for dissecting the enhanced image, and a thinning unit configured for thinning the dissected image; a obtaining unit configured for obtaining a curve point representing a position of a vertebra on the basis of the enhanced, dissected and thinned spinal column image; the processor further includes a generating unit configured for generating a final spinal column curve by using the curve point and a calculating unit configured for calculating a spinal column curvature angle by using an inflection point of the spinal column curve.

17. The device for measuring spinal column curvature according to claim 16, wherein, the enhancing unit includes a device for employing symmetric phase consistency of the spine image and obtaining an enhanced spinal bone feature image; the dissecting unit includes a device for performing double threshold segmentation on the asymmetric phase consistency of the enhanced spinal bone feature image and the spinal column image to obtain a spine segmentation image; the thinning unit includes a device for performing the following steps from top to bottom or bottom to top on the spine segmentation image:

   - find all segmentation lines in the selected row;
   - find the segment with the largest overlap with the previous row among all the segmentation lines found, and compare a distance value between the segment and the previous segment with a threshold $T_h$, if the distance value is less than the threshold $T_h$, the segment is taken as a spine bone cross-sectional area of the current row;
   - repeat the above steps until all rows have been processed.

**18.** The device for measuring spinal column curvature according to claim 16, wherein, in the obtaining unit, the curve point representing the position of the vertebra is realized by finding a position of a minimum region of the gray value of each line of the image in the spine bone cross-sectional area.

**19.** The device for measuring spinal column curvature according to claim 16, wherein, the obtaining unit includes a device for receiving a point marked on the spinal column image provided by an operating sensor and determining it as the obtained curve point representing the position of the vertebra; the obtaining unit further includes a device receiving an adjustment provided by the operating sensor for a coordinate position of the curve point representing the position of the vertebra on the spinal column image.

**20.** The device for measuring spinal column curvature according to anyone of claims 16 - 19, wherein, the generating unit includes a device for using a polynomial fitting of 5th order or more to generate the final spinal column curve on the basis of the curve point.

S1 acquiring a spinal column image by using three-dimensional ultrasound imaging

S2 enhancing, dissecting and thinning the spinal column image

S3 obtaining, on the basis of the enhanced, dissected and thinned spinal column image, a curve point representing a position of a vertebra

S4 generating a final spinal column curve by using the curve point

S5 calculating a spinal column curvature angle by using an inflection point of the spinal column curve

# Fig. 1

acquiring a spinal column image by using three-dimensional ultrasound imaging — S210

On the spinal column image, manually obtaining the curve points representing the position of the vertebra — S220

According to the plurality of curve points obtained by human-computer interaction, these curve points are fitted by a polynomial of 5th order or more to generate a final spine curve — S230

On the basis of the inflection points on the spinal column curve, calculating the spinal column curvature angle — S240

# Fig. 2

Fig. 3a

A1-2:35.0

A1:10.4

A2:16.6

Fig. 3b

acquiring a spinal column image by using three-dimensional ultrasound imaging

S410

On the spinal column image, a curve point representing the position of the vertebra is obtained by a computer aided method

S420

According to the plurality of curve points obtained by human-computer interaction, these curve points are fitted by a polynomial of 5th order or more to generate a final spine curve

S430

On the basis of the inflection points on the spinal column curve, calculating the spinal column curvature angle

S440

Fig. 4

EP 3 449 822 A1

Fig. 5a                    Fig. 5b

16

A1:17.0

A1-2:33.5

A1:16.5

Fig. 5c

acquiring a spinal column image by using three-dimensional ultrasound imaging

S610

On the spinal column image, a curve point representing the position of the vertebra is obtained manually and by a computer aided method

S620

According to the plurality of curve points obtained by human-computer interaction, these curve points are fitted by a polynomial of 5th order or more to generate a final spine curve

S630

On the basis of the inflection points on the spinal column curve, calculating the spinal column curvature angle

S640

Fig. 6

Fig. 7a

Fig. 7b

A1-2:33.4

A1:16.0

A1:16.5

Fig. 7c

Fig. 8a            Fig. 8b            Fig. 8c

Fig. 8d     Fig. 8e     Fig. 8f     Fig. 8g

three-dimensional ultrasound
imaging unit 100

enhancing unit 210

dissecting unit 220

thinning unit 230 —— operating sensor 300

obtaining unit 240

generating unit 250

calculating unit 260

output unit 270

processor 200

Fig. 9

<div align="center">

## INTERNATIONAL SEARCH REPORT

</div>

| International application No. |
| --- |
| **PCT/CN2016/080159** |

**A. CLASSIFICATION OF SUBJECT MATTER**

A61B 5/103 (2006.01) i; A61B 8/00 (2006.01) i; G06T 7/00 (2017.01) i

According to International Patent Classification (IPC) or to both national classification and IPC

**B. FIELDS SEARCHED**

Minimum documentation searched (classification system followed by classification symbols)

A61B, G06T

Documentation searched other than minimum documentation to the extent that such documents are included in the fields searched

Electronic data base consulted during the international search (name of data base and, where practicable, search terms used)

CNABS, CNTXT, CNKI, IEEE, EPODOC, WPI, EPTXT, USTXT, WOTXT: protrude, spine, vertebra+, scoliosis, bone, skeleton, deform+, bend+, curv+, inflect+, enhanc+, segment+, thin+, refin+, angle, imag+, picture?, phase, dot?, point

**C. DOCUMENTS CONSIDERED TO BE RELEVANT**

| Category* | Citation of document, with indication, where appropriate, of the relevant passages | Relevant to claim No. |
| --- | --- | --- |
| Y | CN 102497821 A (THE HONG KONG POLYTECHNIC UNIVERSITY), 13 June 2012 (13.06.2012), description, paragraphs 10-79, and figures 1-10 | 1-2, 6-12, 14-16, 18-20 |
| Y | CN 1969756 A (SIEMENS MEDICAL SOLUTIONS USA, INC.), 30 May 2007 (30.05.2007), description, page 4, paragraph 1 to page 9, paragraph 2, and figures 2-15 | 1-2, 6-12, 14-16, 18-20 |
| A | CN 1449721 A (TOSHIBA CORPORATION), 22 October 2003 (22.10.2003), the whole document | 1-20 |
| A | CN 103417243 A (TELEFIELD MEDICAL IMAGING LIMITED), 04 December 2013 (04.12.2013), the whole document | 1-20 |
| A | US 2014303522 A1 (TOYO UNIVERSITY), 09 October 2014 (09.10.2014), the whole document | 1-20 |
| A | US 2007242869 A1 (EASTMAN KODAK CO.), 18 October 2007 (18.10.2007), the whole document | 1-20 |

☒ Further documents are listed in the continuation of Box C.     ☒ See patent family annex.

| | |
| --- | --- |
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 22 January 2017 (22.01.2017) | **03 February 2017 (03.02.2017)** |

| Name and mailing address of the ISA/CN:<br>State Intellectual Property Office of the P. R. China<br>No. 6, Xitucheng Road, Jimenqiao<br>Haidian District, Beijing 100088, China<br>Facsimile No.: (86-10) 62019451 | Authorized officer<br><br>**WEI, Na**<br><br>Telephone No.: (86-10) **62089912** |

Form PCT/ISA/210 (second sheet) (July 2009)

<table>
<tr><td colspan="2" align="center"><b>INTERNATIONAL SEARCH REPORT</b></td><td>International application No.<br><br><b>PCT/CN2016/080159</b></td></tr>
</table>

**C (Continuation).    DOCUMENTS CONSIDERED TO BE RELEVANT**

| Category* | Citation of document, with indication, where appropriate, of the relevant passages | Relevant to claim No. |
|---|---|---|
| A | CN 1336621A (LUCENT TECHNOLOGIES INC.), 20 February 2002 (20.02.2002), the whole document | 1-20 |

Form PCT/ISA/210 (continuation of second sheet) (July 2009)

## INTERNATIONAL SEARCH REPORT
Information on patent family members

International application No.

**PCT/CN2016/080159**

| Patent Documents referred in the Report | Publication Date | Patent Family | Publication Date |
|---|---|---|---|
| CN 102497821 A | 13 June 2012 | AU 2010278526 B2 | 18 February 2016 |
| | | JP 5849048 B2 | 27 January 2016 |
| | | CN 102497821 B | 08 April 2015 |
| | | JP 2013500089 A | 07 January 2013 |
| | | US 2011021914 A1 | 27 January 2011 |
| | | EP 2459073 A4 | 27 August 2014 |
| | | US 8900146 B2 | 02 December 2014 |
| | | EP 2459073 A1 | 06 June 2012 |
| | | AU 2010278526 A1 | 08 March 2012 |
| | | CA 2769150 A1 | 03 February 2011 |
| | | WO 2011012055 A1 | 03 February 2011 |
| CN 1969756 A | 30 May 2007 | DE 102006041619 A1 | 19 April 2007 |
| | | US 7715605 B2 | 11 May 2010 |
| | | US 2007055178 A1 | 08 March 2007 |
| | | JP 2007069007 A | 22 March 2007 |
| CN 1449721 A | 22 October 2003 | JP 4070493 B2 | 02 April 2008 |
| | | CN 100488451 C | 20 May 2009 |
| | | US 2003215122 A1 | 20 November 2003 |
| | | US 7231073 B2 | 12 June 2007 |
| | | JP 2003290217 A | 14 October 2003 |
| CN 103417243 A | 04 December 2013 | CN 103417243 B | 27 May 2015 |
| US 2014303522 A1 | 09 October 2014 | WO 2013081030 A1 | 06 June 2013 |
| | | JPWO 2013081030 A1 | 27 April 2015 |
| US 2007242869 A1 | 18 October 2007 | WO 2007126667 A2 | 08 November 2007 |
| | | EP 2005392 A2 | 24 December 2008 |
| CN 1336621 A | 20 February 2002 | EP 1176544 A1 | 30 January 2002 |
| | | JP 2002109534 A | 12 April 2002 |
| | | CA 2349789 A1 | 27 January 2002 |
| | | KR 20020010523 A | 04 February 2002 |

Form PCT/ISA/210 (patent family annex) (July 2009)